# EUROPEAN PATENT SPECIFICATION

(11) **EP 2 363 087 B1**
(45) Date of publication and mention of the grant of the patent: **07.05.2014**
(21) Application number: 10155541.5
(22) Date of filing: 04.03.2010
(51) Int. Cl.: A61F 2/42

(54) **Fastening system for prostheses**
Befestigungssystem für Prothesen
Système de fixation pour prothèses

(43) Date of publication of application: 07.09.2011
(73) Proprietor: Younger, Alastair, Vancouver, BC V6Z2A5 (CA)
(72) Inventor: Younger, Alastair, Vancouver, BC V6Z2A5 (CA)
(74) Representative: Perani, Aurelio

(56) References cited:
- EP-A1- 1 097 680
- WO-A2-2009/023666
- US-A- 5 360 452
- US-A1- 2008 109 081
- US-B1- 6 663 669

## Description

### Technical Field

This invention relates to prosthetic implants, and in particular to systems for fastening prosthetic implants such as ankle prostheses.

### Background

Cementless ankle prostheses for total ankle replacement surgeries are known. For example, the Scandinavian Total Ankle Replacement (S.T.A.R.⁷) system by Small Bone Innovations, Inc. is a three piece prosthesis including a talar component, mobile bearing and tibial component. Like many known systems, the S.T.A.R.⁷ system relies on relatively large projections to fasten the prosthesis to bone. The S.T.A.R.⁷ system, for example, features a fin on the talar implant that inserts caudally into a cut made in the talar dome, and two barrels oriented in the anterior/posterior direction on the tibial implant that insert into drilled holes in the tibia. Disadvantages of prostheses having relatively large projections include requiring a significant amount of bone removal and compromising blood supply in the bone.

Before an ankle prosthesis can be implanted, preparatory cuts in the talus and tibia must be made to facilitate positioning of the prosthesis. Typically, the preparatory cuts are not precisely complementary to the bone-contacting surfaces of the prosthesis. This imprecision may be due for example to differences in bone density across the section of the bone being cut. Imprecise preparatory cuts can result in imprecise fastening of the prosthesis to bone, leading to subsequent subsidence, loosening, and/or aseptic failure.

Also before implantation, a drilling template is typically placed over the pre-cut bone surface to guide the drilling of fastener holes in the bone surface. The drilling template is then removed, the actual prosthesis is positioned on the bone surface, and fasteners are inserted through the fastener holes of the prosthesis into the bone to fasten the prosthesis. Typically, however, the alignment of the drilling template on the bone is not identical to the alignment of the actual prosthesis on the bone, a difference due for example to the bone-contacting surface of the drilling template not corresponding precisely in profile to the bone-contacting surface of the prosthesis. The resulting misalignment between the drilled holes in the prepared bone surface and the holes in the actual prosthesis positioned on the prepared bone surface can also result in imprecise fastening of the prosthesis to bone. This misalignment can be further exacerbated by imprecise preparatory cuts in the bone as described the bone as described above. **Another example of fastening system for prostheses in given by document** US2008/109081**, which substantially discloses the preamble of claim 1.** Cementless prosthetic implants that provide less intrusive but more precise and stable fastening to bone are therefore desired.

### Summary

The ankle prosthesis according to the invention is as specified in claim 1.

Further aspects of the invention are exposed in the dependent claims.

A jig for drilling holes for prosthesis fasteners with a cannulated drill can be used with a prosthesis according to the invention. The jig includes: a prosthetic implant with a threaded fastener hole; a guide with a longitudinal bore and an externally threaded distal end for threadingly engaging the fastener hole; a cannula for insertion in the longitudinal bore of the guide, the cannula having an longitudinal bore; and a guidewire for insertion in the longitudinal bore of the cannula, the guidewire guiding the cannulated drill. The longitudinal bore of the cannula or the guide may be eccentric. The distal end of the guide may be frustoconical. The guide may have a slotted proximal end for engagement with a T-handle.

A prosthesis according to the invention can be used in a method of fastening a prosthesis to bone. The method includes the steps of:
(a) precutting the bone to provide a bone surface complementary to a bone-contacting surface of the prosthesis;
(b) positioning the prosthesis on the bone surface;
(c) implanting a threaded guidewire into a location of bone through a fastening hole of the prosthesis positioned on the bone;
(d) drilling a drill hole in the bone through the fastening hole using a cannulated drill guided by the threaded guidewire;
(e) inserting through the fastening hole and into the drill hole a fastener comprising a locking head and an unthreaded elongated body; and
(f) locking the head of the fastener in the fastening hole to fasten the prosthesis to the bone.

Step (b) may involve overdrilling the drill hole to prevent the fastener from backing out of the drill hole. In step (b) the diameter of the drill hole may be less than the diameter of the unthreaded elongated body of the fastener, so the drill hole by scratch fit. Step (c) may include implanting the threaded guidewire eccentric in relation to the fastening hole. Step (d) may include drilling the drill hole eccentric in relation to the fastening hole. Step (f) may include pulling the prosthesis in a direction corresponding to the eccentricity of the drill hole.

### Brief Description of Drawings

In drawings which show non-limiting embodiments of the invention:
Figure 1 is a perspective view of an ankle prosthesis ;
Figure 2 is a top view of the talar implant illustrated in Figure 1;
Figure 3 is a side view of the talar implant illustrated in Figure 1;
Figure 4 is a front view of the talar implant illustrated in Figure 1;
Figure 5 is a top view of the tibial implant illustrated in Figure 1;
Figure 6 is a side view of the tibial implant illustrated in Figure 1;
Figure 7 is a front view of the tibial implant illustrated in Figure 1;
Figure 8 is a perspective view of an ankle prosthesis;
Figure 9 is a top view of the talar implant illustrated in Figure 8;
Figure 10 is a side view of the talar implant illustrated in Figure 8;
Figure 11 is a front view of the talar implant illustrated in Figure 8;
Figure 12 is a top view of the tibial implant illustrated in Figure 8;
Figure 13 is a side view of the tibial implant illustrated in Figure 8;
Figure 14 is a front view of the tibial implant illustrated in Figure 8;
Figure 15 is a perspective view of a fastening hole;
Figure 16 is a top view of the fastening hole illustrated in Figure 15;
Figure 17 is a side view along plane A-A in Figure 16;
Figure 18 is a perspective view of a fastening hole ;
Figure 19 is a top view of the fastening hole illustrated in Figure 18;
Figure 20 is a side view along plane A-A in Figure 19;
Figure 21 is a perspective view of a fastening hole according to one embodiment of the invention;
Figure 22 is a top view of the fastening hole illustrated in Figure 21;
Figure 23 is a side view along plane A-A in Figure 22;
Figure 24 is a perspective view of a fastening hole according to one embodiment of the invention;
Figure 25 is a top view of the fastening hole illustrated in Figure 24;
Figure 26 is a side view along plane A-A in Figure 25;
Figures 27A to 27C are side views of screw lock fasteners according to various embodiments of the invention;
Figure 28A to 28C are side views of taper lock fasteners according to various embodiments of the invention;
Figures 29 to 32 are cutaway side views illustrating the steps in fastening an implant to bone ;
Figures 33 to 36 are cutaway side views illustrating the steps in fastening an implant according to the invention to bone (Figure 33b is a cross sectional view along plane B-B in Figure 33a);
Figure 37 is a perspective view of a talar implant ;
Figure 38 is a front view of the talar implant illustrated in Figure 37;
Figure 39 is a perspective view of a talar implant; and
Figure 40 is a view of a talar implant .

### Description

Throughout the following description, specific details are set forth in order to provide a more thorough understanding of the invention. However, the invention may be practiced without these particulars. In other instances, well known elements have not been shown or described in detail to avoid unnecessarily obscuring the invention. Accordingly, the specification and drawings are to be regarded in an illustrative, rather than a restrictive, sense.

Throughout this specification, the terms "proximal" and "distal" refer to positions respectively closer to and further from the surgeon.

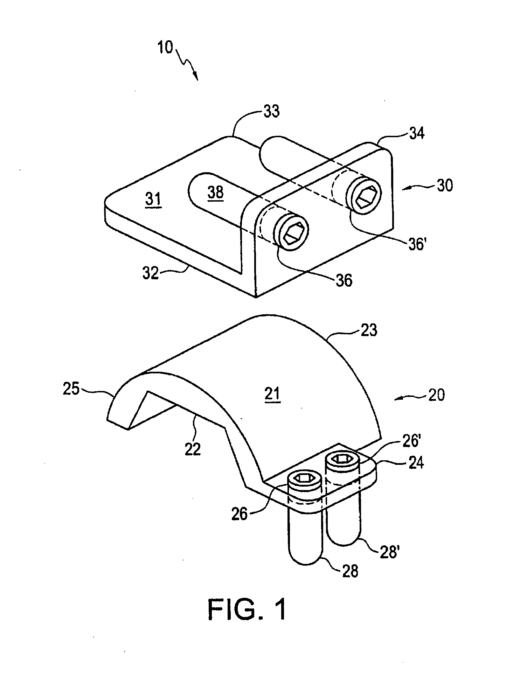

Figure 1 shows a left ankle prosthesis 10. A right ankle prosthesis would be a mirror image of left ankle prosthesis 10. Ankle prosthesis 10 is a cementless three-piece ankle prosthesis that includes a talar implant 20 for fastening to the talar dome, a tibial implant 30 for fastening to the bottom of the tibia, and a mobile bearing (not shown) moveable between the talar implant 20 and tibial implant 30. Talar implant 20 and tibial implant 30 may be made of medical grade metal (e.g. titanium) and/or metal alloy (e.g. chromium/cobalt), or other similar bio-compatible materials. The mobile bearing may be made of medical grade ceramic, plastic (e.g. ultra high molecular weight polyethylene) or other similar bio-compatible materials.

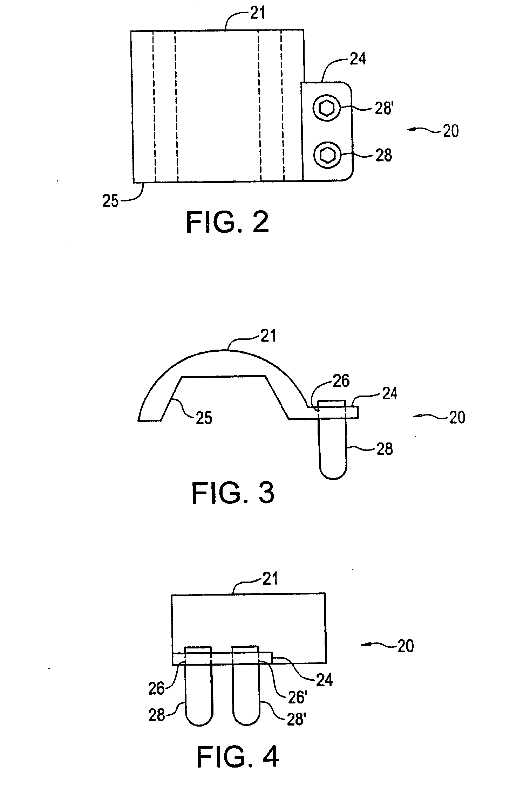

As shown in Figures 1 to 4, talar implant 20 has a generally C-shaped body 21 with a bottom surface 22 and a top surface 23. Body 21 may also be dome-shaped. Bottom surface 22 is concave and shaped to fit over the talar dome. Bottom surface 22 may be provided with a coating to accelerate bone ingrowth for aiding fixation. The talar dome may be pre-cut to provide a complementary surface for registering with bottom surface 22. For example, the talar dome would have a trapezoidal cut on top of which bottom surface 22 would register. Bottom surface 22 may be provided with one or more spikes (not shown) for anchoring talar implant 20 to the talus. Top surface 23 is in free frictional sliding contact with a complementary shaped bottom surface of the mobile bearing.

Body 21 may include a flange 24 that extends forward from the medial side of the anterior end of body 21. Flange 24 is configured to rest substantially flat against the neck of the talus. The neck of the talus may be pre-cut to provide a flat surface for flange 24 to rest against.

Posterior portion 25 of implant 20 extends downward to about the plane of flange 24. In other implants, posterior portion 25 may not extend as far downward as, or may extend further downward below, the plane of flange 24.

Flange 24 includes fastening holes 26, 26'. As described further below, fasteners 28, 28' are locked vertically in fastening holes 26, 26' after insertion into underlying bone tissue to fasten talar implant 20 to the talus.

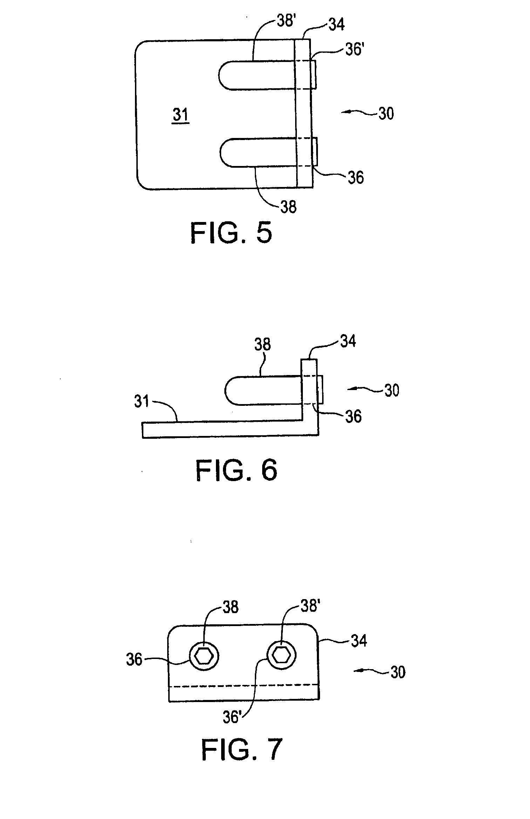

As shown in Figures 1 and 5 to 7, tibial implant 30 has a plate 31 having a bottom surface 32 and a top surface 33. Plate 31 extends along the sagittal length of the tibia and bears against the lateral portion of the lower end of the tibia. Bottom surface 32 is in free frictional sliding contact with a complementary shaped top surface of the mobile bearing. Top surface 33 may be provided with a coating to accelerate bone ingrowth from the lower end of the tibia to aid fixation. The lower end of the tibia may be pre-cut to provide a flat surface for plate 31 to bear against. A flange 34 extends upwards from the anterior end of plate 31. Flange 34 includes fastening holes 36, 36'. Fasteners 38, 38' are locked horizontally in fastening holes 36, 36' after insertion into underlying bone tissue to fix tibial implant 30 to the tibia. Fasteners 38, 38' may be oriented parallel to the medial border of the tibia in the transverse plane.

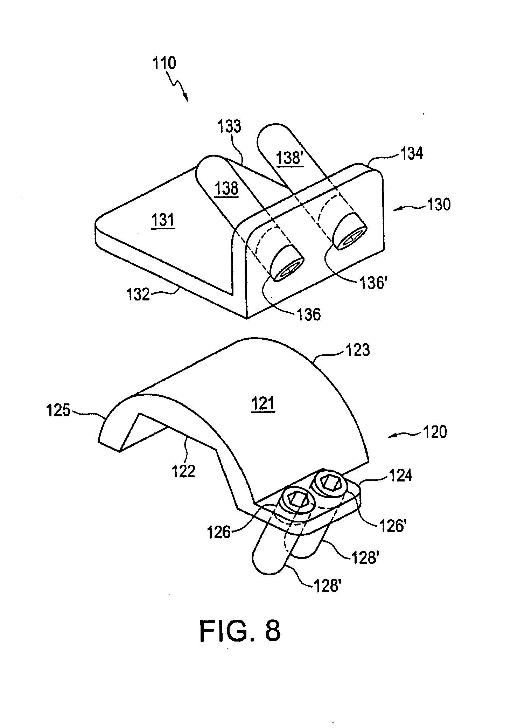

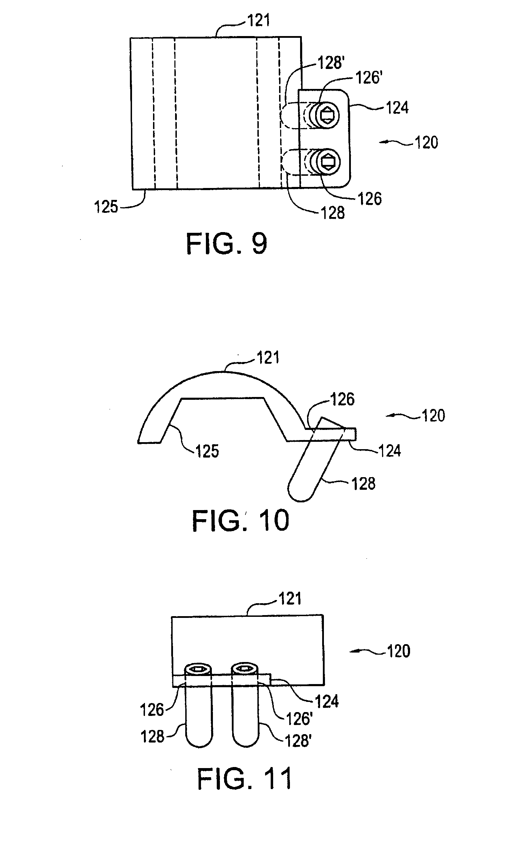

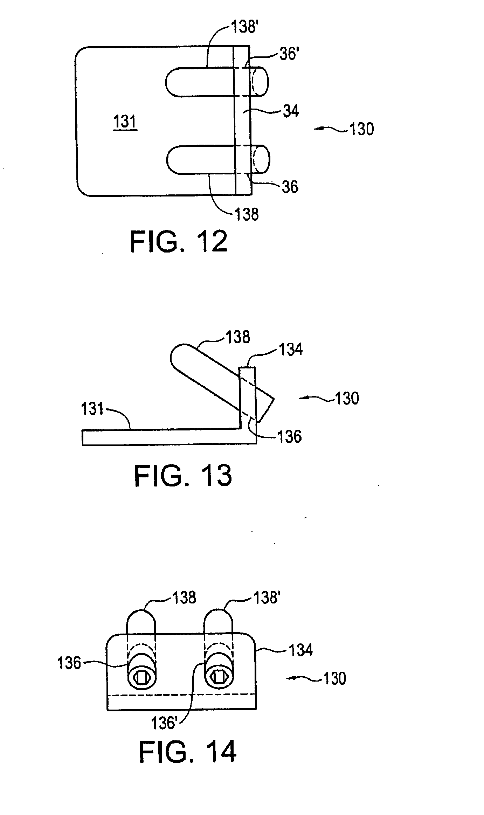

Figures 8-14 shows ankle prosthesis 110. Ankle prosthesis 110 has features identical to ankle prosthesis 10 except that for both talar implant 120 and tibial implant 130, the fastening holes are designed for angled insertion of fasteners. Unless otherwise stated, the angles of the fasteners as discussed in this specification relate to angles in the sagittal plane. For talar implant 120, fasteners 128, 128' are locked in fastening holes 126, 126' in flange 124 at an acute angle in the posterior direction. The angle at which fasteners 128, 128' penetrate the talus may be parallel to the angle of posterior extension 125 of body 121. For tibial implant 130, fasteners 138, 138' are locked in fastening holes 136, 136' at an upward angle in the posterior direction. Angled upward insertion of fasteners 138, 138' allows the fasteners to be anchored in the compact bone of the diaphysis of the tibia, resulting in more secure fastening compared to horizontal insertion of the fasteners into the spongy bone of the epiphysis of the tibia.

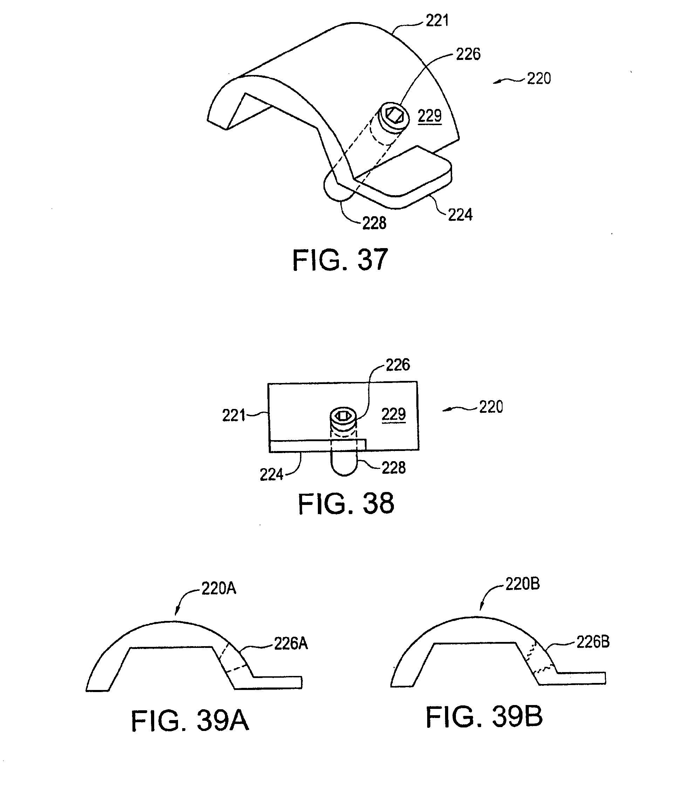

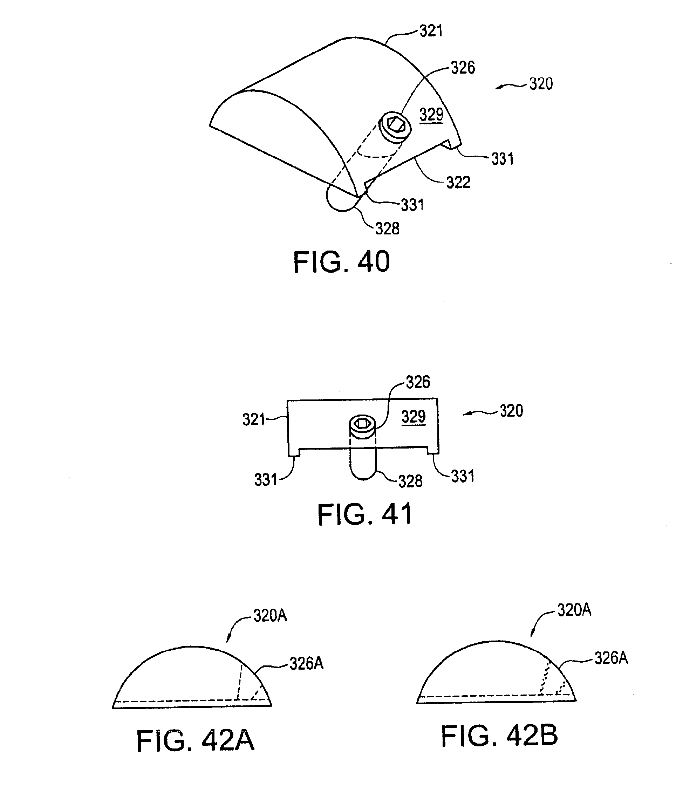

Figures 37 and 38 show another talar implant 220 provided with at least one fastening hole 226 for receiving fastener 128 in an upper anterior face 229 of body 221 at an acute angle. Body 221 may be C-shaped or dome-shaped with a concavity, for example. Fastener 228 and fastening hole 226 correspond in form and function to fasteners 128, 128' and fastening holes 126, 126' of talar implant 120. In some implants, such as the one illustrated in Figures 37 and 38, talar implant 220 may have an anterior flange 224, and anterior flange 224 may be provided with at least one fastening hole (not shown) for receiving at least one fastener as described above for talar implants 20, 120. Upper anterior surface 229 may not articulate with the mobile bearing. Figures 39A and 39B show examples of talar implant 220, namely talar implants 220A, 220B having taper lock fastening hole 226A and screw lock fastening hole 226B respectively. Other talar implants could be provided in a modified form for bone loss and revision surgeries. Instead of a C-shaped body or a dome- shaped body with a concavity, the talar implant may be provided as a solid dome having a base to bear against a pre-cut portion of the talar dome as shown in Figures 40 and 41. Talar implant 320 shown in Figure 40 has a solid dome-shaped body 321 with a substantially flat bottom surface 322. By "substantially" flat bottom surface it is understood that bottom surface 322 may be completely flat, or may have, for example, one or more small projections (e.g. spikes) or indentations to facilitate fastening of the implant to bone or facilitate registration between the implant and bone. At least one fastener hole 326 receives at least one fastener 328 at an acute angle through an upper anterior surface 329 of body 321 to bottom surface 322 so that fastener 328 penetrates through body 321 to fasten talar implant 320 to bone. Some talar implants 320 may have an anterior flange (not shown) provided with at least one fastening hole for receiving at least one fastener as described above for talar implants 20, 120. Upper anterior surface 329 may not articulate with the mobile bearing. As shown in Figure 41, talar implant 320 may be provided with lateral guide rails 331 extending in the anterior/posterior direction to facilitate positioning of talar implant 320 on the pre-cut talus bone. Figures 42A and 42B show examples of talar implant 320, namely talar implants 320A, 320B having taper lock fastening hole 326A and screw lock fastening hole 326B respectively.

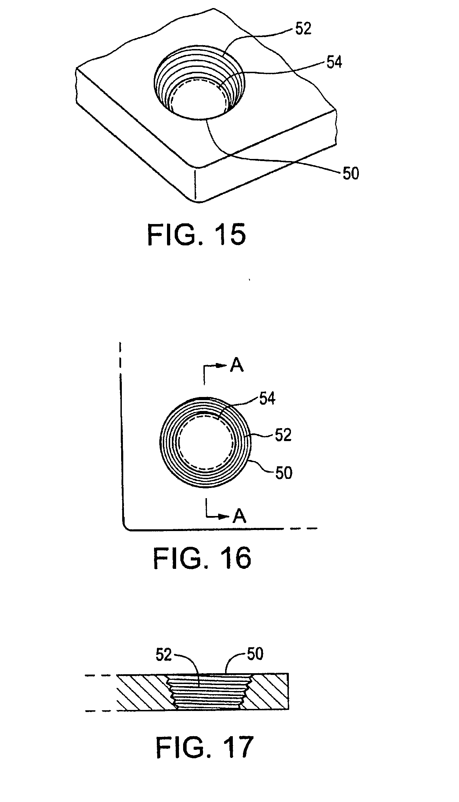

Figures 15 to 17 show fastening hole 50, an example of fastening holes 26, 26', 36 and 36'. Fastening hole 50 is configured for "neutral" locking of a fastener. Fastening hole 50 is frustoconical (as best shown in Figure 17) and has internally spiralling threads 54 for receiving a frustoconical threaded head of a fastener. As described below, drill hole 58 must be made in the underlying bone before the fastener can be inserted. Drill hole 58 is centered with respect to fastening hole 50. The diameter of drill hole 58 may be somewhat less than the diameter of the fastener to provide an interference or "scratch" fit. In other implants, the diameters of the drill hole and fastener may be substantially the same. The fastener is locked in as the thread of the fastener head is threaded into fastening hole 50.

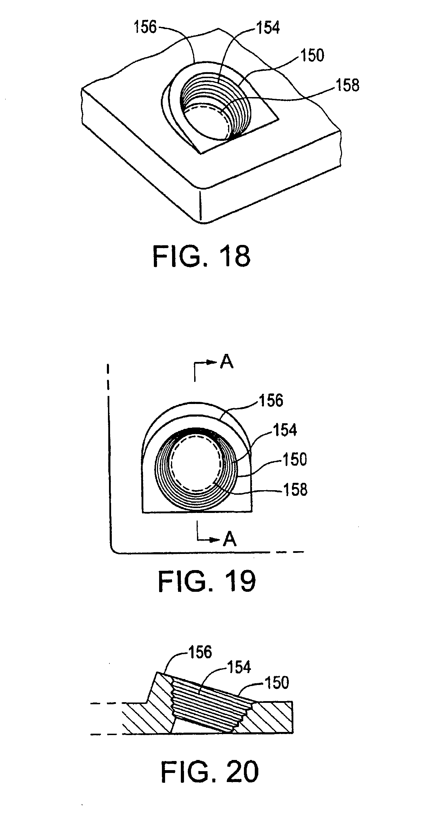

Figures 18 to 20 show fastening hole 150, an example of fastening holes 126, 126', 136, 136', 226, and 326. Fastening hole 150 is configured for neutral locking of a fastener at an acute angle to the face of the implant. Fastening hole 150 is frustoconical and has internally spiralling threads 154. Drill hole 158 made in the underlying bone is centered with respect to fastening hole 150. Like drill hole 58, the diameter of drill hole 158 may be somewhat less than the diameter of the fastener to provide a scratch fit. Fastening hole 150 is also partly defined by a ramped portion 156 projecting from the face of the implant to accommodate complete locking of the head of the fastener within fastening hole 150. Other implants may provide sufficient depth (i.e., be sufficiently thick) so as to not to require a ramped portion to achieve complete locking of the fastener at an angle within the implant.

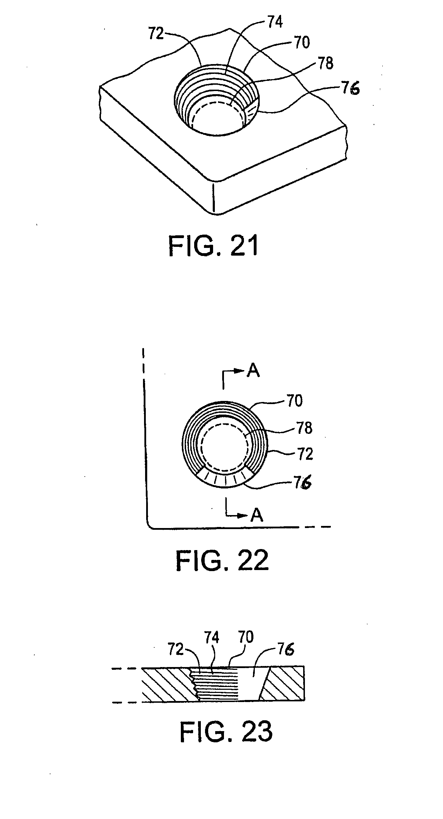

Figures 21 to 23 show fastening hole 70, an embodiment of fastening holes 26, 26', 36, 36'. Fastening hole 70 is configured for "compression" locking of a fastener. Fastening hole 70 is frustoconical and has a threaded arc section 72 having internally spiralling threads 74 for receiving the threaded head of a fastener. Unlike fastening hole 50, fastening hole 70 has an unthreaded arc section 76 where internally spiralling threads 74 are absent. In some embodiments, such as the embodiment illustrated, the unthreaded arc section may extend from the proximal end to the distal end offastening hole 70; in other embodiments, where the fastening hole is also frustoconical, the unthreaded arc section may only occupy a distal portion of an arc of fastening hole 70. Because internally spiralling threads 74 are absent at unthreaded arc section 76, the interior radius at the distal end of fastening hole 70 along unthreaded arc section 76 is slightly greater than the interior radius at the distal end of fastening hole 70 along threaded arc section 72 (as best shown in Figure 22). Drill hole 78 is made in underlying bone before insertion of the fastener. Like drill hole 58, the diameter of drill hole 78 may be somewhat less than the diameter of the fastener to provide a scratch fit. The slightly greater interior radius of the distal end of fastening hole 70 along unthreaded arc section 76 allows for eccentric placement of drill hole 76. In particular, drill hole 78 is not centered with respect to fastening hole 70 and is instead shifted slightly toward unthreaded arc section 76 by, for example, 0.5 to 1.0 mm (as shown best in Figure 22). As a result, as the fastener is being locked into fastening hole 70 (i.e., as the threaded head of the fastener is matingly received in the fastening hole 70), the elongated body of the fastener inserted into the bone acts as the resistance for levering the implant against underlying bone lying in the general direction of the unthreaded arc section 76.

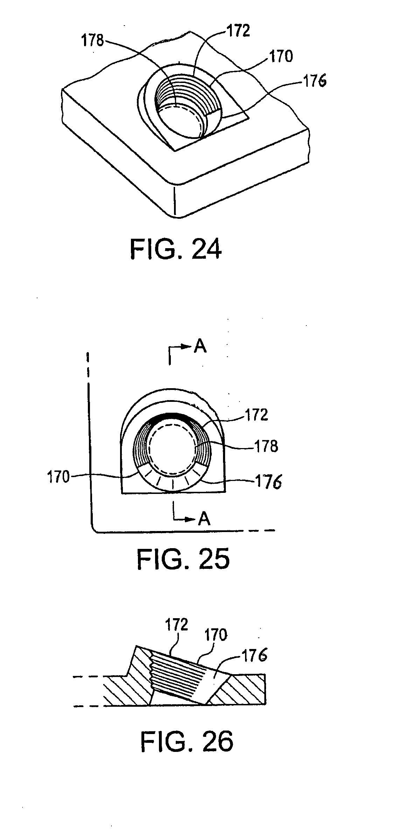

Figures 24 to 26 show fastening hole 170, an alternative embodiment of fastening holes 126, 126', 136, and 136'. Fastening hole 170 is configured for compression locking of a fastener at an acute angle to the face of the implant. Similar to fastening hole 150, fastening hole 170 has a ramped portion 176 to accommodate complete locking of a head of a fastener within fastening hole 170 at an angle. Otherwise, fastening hole 170 has features and functions analogous to fastening hole 70. For example, fastening hole 170 has a threaded arc section 172 with internally spiralling threads 174, an unthreaded arc section 176 where internally spiralling threads 174 are absent, and an eccentrically placed drill hole 178 having a diameter somewhat less than the diameter of the fastener to provide a scratch fit.

For compression locking of a talar implant, the unthreaded arc section may be provided on the anterior side of the fastening hole. As the fastener head is locked in to the fastener hole, the posterior extension of the implant is pulled slightly in an anterior direction against the posterior portion of the talus below the implant. The precise direction of the pulling force depends on the angle of insertion of the fastener. For talar implant 20 with flat fastening holes, the pulling force would be horizontal in the anterior direction for secure fixation. Even further secure fastening is achieved with talar implant 120 with angled fastening holes, wherein the pulling force would be at an angle, in a downward and anterior direction.

For compression locking with a tibial implant, the unthreaded arc section may be provided on the upper side of the fastening hole. As the fastener is locked in, the plate is pulled slightly in an upward direction against the bottom of the tibia. Again, the precise direction of the pulling force depends on the angle of insertion of the fastener. For tibial implant 30 with flat fastening holes, the pulling force would be directly upward for secure fixation. Even further secure fastening is achieved with tibial implant 130 with angled fastening holes, wherein the pulling force would be in an upward and anterior direction.

In other fastening holes, whether for neutral or compression locking, the fastening hole(s) and corresponding fastener head(s) may for example be unthreaded (e.g. for taper locking) rather than threaded, and/or cylindrical rather than frustoconical.

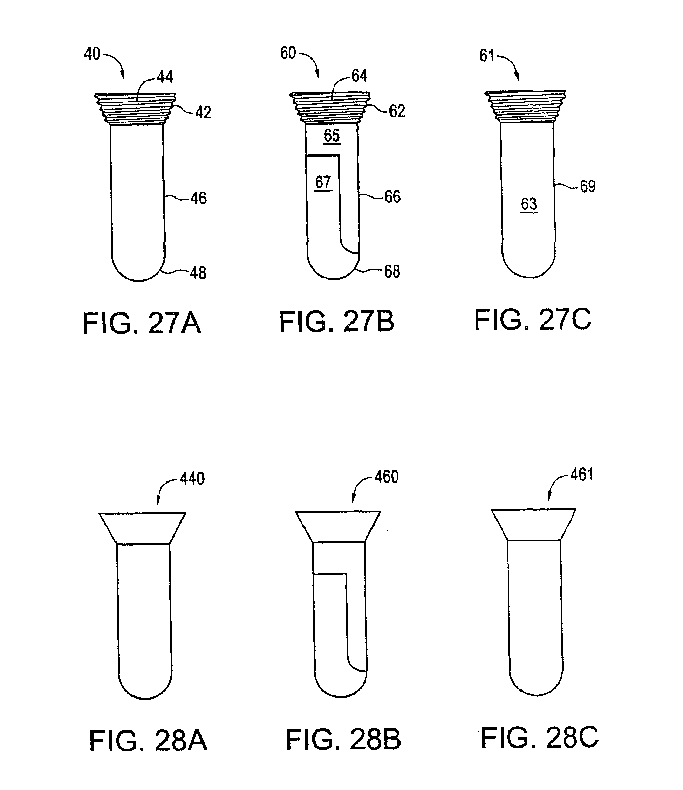

Fasteners for use in the present invention, including fasteners 28, 28', 38, 38', 128, 128', 138, 138', 228, and 328 may be a peg, spike or the like of different shapes with different locking means such as a taper lock, barb lock, screw lock, expansion lock, secondary lock or any other known type of lock for forming a secure, locking connection between a fastener and a fastening hole. Fastener 40 shown in Figure 27A is an example embodiment of a screw lock fastener. Fastener 40 has a head 42 having sides with externally spiralling threads 44. Head 42 may have a top (not shown) having a suitably shaped depression or protrusion for mating connection with a screwdriver, drill, or other means for locking fastener 40 into the fastening hole. Fastener 40 also has an unthreaded elongated body 46 that includes a rounded or hemispherical end portion 48. Body 46 has a smooth surface.

Figure 27B shows fastener 60, another embodiment of a fastener with a screw lock. Fastener 60 is similar to fastener 40, with head 62 having sides with externally spiralling threads 64, and top of head 62 having features suitable for locking fastener 60 into the fastening hole. Unlike fastener 40, elongated body 66 of fastener 60 has at least one region with a smooth surface 65 and at least one region with a non-smooth surface 67. Non-smooth surface 67 may be any type of coating or surface treatment adaptable or suitable for ingrowth of boney tissue. Non-smooth surface 67 may for example be a porous surface formed by hydroxyapatite coating, cement coating, grit blasting or other known methods.

Smooth surface 65 may occupy a region encircling body 66 adjacent head 64 and a region extending along the length of elongated body 66 toward end portion 68. The remaining surface of body 66 may be occupied by non-smooth surface 67. In other embodiments, the elongated body of the fastener may have a different distribution of smooth and non-smooth surfaces, or may have a completely non-smooth surface.

When fastener 60 is in use with fastening holes such as fastening holes 70, 170 for compression locking, smooth surface 65 extending along elongated body 66 is aligned with and bears against the unthreaded arc section of the fastening hole during insertion. This alignment, which effectively shifts fastener 60 off center in the direction of the unthreaded arc section, in turn provides a small gap between the non-smooth surface 67 of fastener 60 and the distal end of the threaded arc section of the fastening hole. This small gap ensures minimal damaging contact to non-smooth surface 67 as fastener 60 glides through the fastening hole during insertion into bone.

Figure 27C shows fastener 61, a further embodiment of a fastener with a screw lock. Fastener 61 is identical to fastener 40 except that elongated body 69 has a completely non-smooth surface 63.

Figures 28A, 28B, and 28C show fasteners 440, 460, 461 which are identical in features and functions to fasteners 40, 60, 61 respectively except that fasteners 440, 460, 461 employ taper locks and therefore have non-threaded frustoconical heads.

The dimensions of the fasteners described herein may vary. For example, for the elongated body of the fasteners, the diameter may be 4 mm, and the length may range from 12 to 30 mm. The shape and size of the fastener head of the fasteners would be complementary to the shape and size of the corresponding fastener hole. The fasteners may be compatible with small or large fragment surgical instruments.

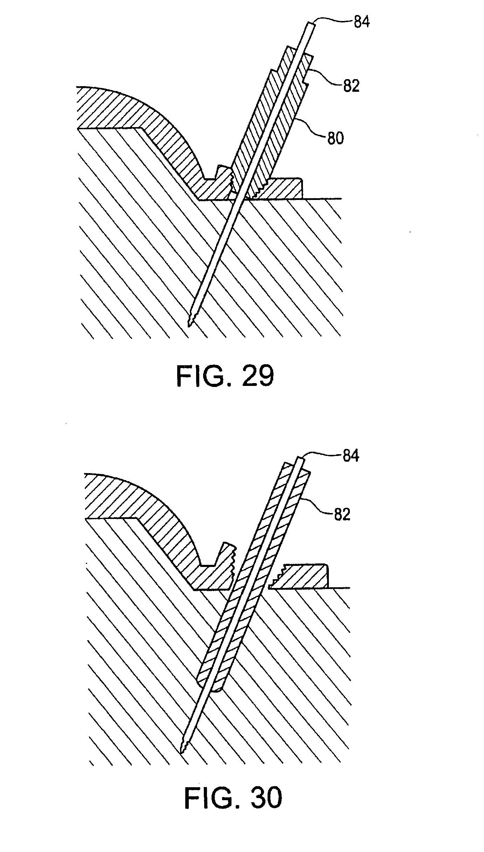

The present invention avoids the use of a drilling template, and the problems associated therewith, by incorporating use of the final implant as part of the drilling jig. Figures 29 to 32 illustrate a jig for preparing a drill hole for neutral locking fastening of a talar implant to the talus bone. In these figures, the talar implant may be talar implant 120, the fastening hole may be 50, 150, and the fastener may be 40, 60, for example. As shown in Figure 29, a guide 80 with an externally threaded distal end is threaded into the fastening hole. Threaded guide 80 may for example be about 5 cm in length with a 2 mm longitudinal bore. A T-handle (not shown) may be connected to the proximal end 82 to assist in threading threaded guide 80 into the fastening hole. Guidewire 84 is then passed through threaded guide 80 and screwed or drilled into the bone. Guidewire 84 may for example be 2 mm in diameter.

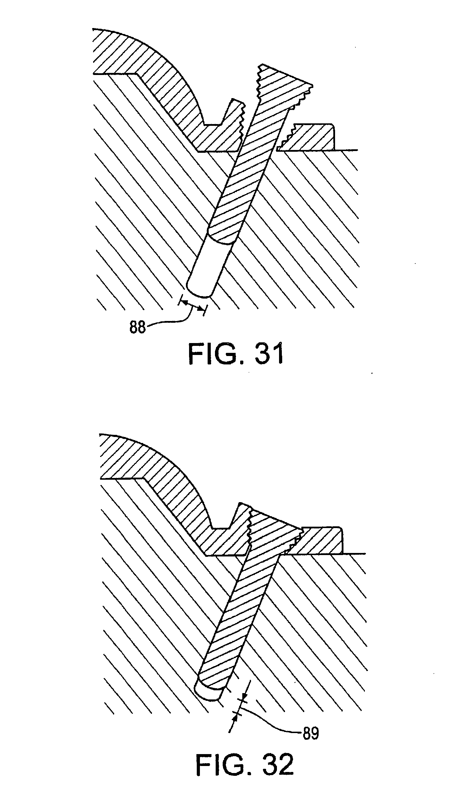

As shown in Figure 30, threaded guide 80 is then removed, and a cannulated drill 86, guided by guidewire 84, is used to drill a hole into the bone. As shown in Figures 30 and 31 and mentioned above, drill 86 may drill a hole with a diameter 88 (e.g. 3.9 mm) slightly less than the diameter of the fastener's elongated body (e.g. 4.0 mm) to provide a scratch fit of the fastener. As shown in Figure 32, the drill hole may be drilled deeper than the length of the fastener by an overdrill depth 89 to prevent the fastener from backing out of the drill hole over time. Overdrill depth 89 may, for example, be 4 to 6 mm. Since the implant is already in position (i.e., there is no need to remove a drilling template and replace it with the implant), the fastener may now be pushed or impacted in, and then locked in to the fastening hole with a screwdriver or drill, for example.

Similar steps as those illustrated in Figures 29 to 32 would be involved for drilling a fastener hole into a tibial bone for neutral locking of the fastener, for example for tibial implant 130 (not shown).

Figures 29 to 32 illustrate drilling for neutral locking with angled fastening; drilling for vertical/horizontal fastening (e.g. for implants 20, 30) may not require a threaded guide or guidewire and may instead be simply performed using an uncannulated drill and the fastening hole itself as a visual guide.

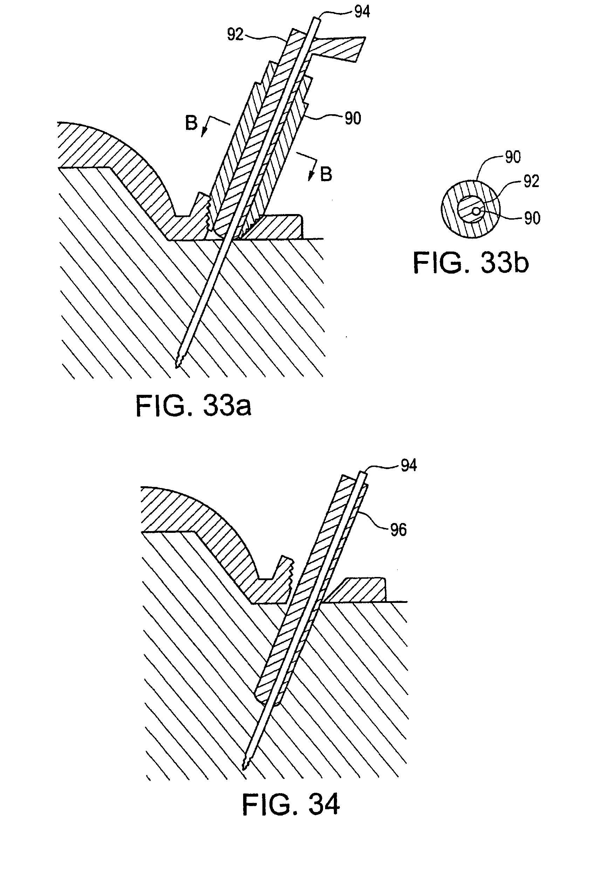

Figures 33 to 36 illustrate a jig for compression locking of a fastener according to an embodiment of the invention. Again, the use of a drilling template is avoided by adapting the actual prosthesis as a drilling guide. In these figures, the implant may be implant 20, 120, 220, 320, the fastening hole may be 70, 170, and the fastener may be 40, 60, for example. As shown in Figure 33, a threaded guide 90 is threaded into the fastening hole, again optionally with the aid of a T-handle connected to the distal end of threaded guide 90. Threaded guide may for example be 4 cm in length with a 3 mm longitudinal bore. A cannula 92 with a guidewire 94 positioned in an eccentric longitudinal bore (as best shown in Figure 33a) is slid into threaded guide 90. Cannula 92 may for example be 5 cm in length, 3 mm in outer diameter, and be provided with a handle at the proximal end. The eccentric positioning is such that the placement of the guidewire is shifted toward the unthreaded section of the fastening hole. Guidewire 94 is then screwed or drilled into the bone. The longitudinal bore of the threaded guide, rather than the cannula, may be eccentric.

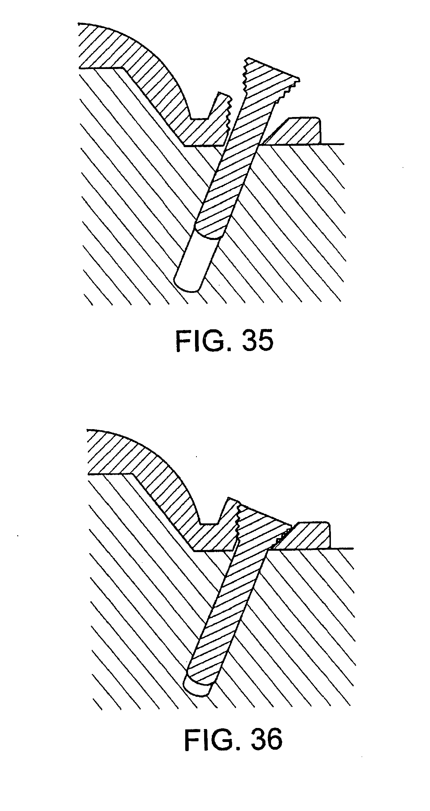

As shown in Figure 34, threaded guide 90 and cannula 92 are then removed, and a cannulated drill 96, guided by guidewire 94, is used to drill a hole into the bone. As shown in Figure 35, the resulting drill hole is shifted toward the unthreaded arc section of the fastening hole. Similar to the neutral locking option: the diameter of the drill hole 98 may be slightly less than the diameter of the fastener to provide a scratch fit; the drill hole may be drilled deeper than the length of the fastener by an overdrill depth 99 of for example 4 to 6 mm to ensure the fastener does not back out; and since the implant is already in position, the fastener may then be pushed or impacted in, and then locked in to the fastening hole with a screwdriver or drill.

Similar steps as those illustrated in Figures 33 to 36 would be involved for drilling a fastener hole into a tibial bone for compression locking of the fastener, for example for tibial implant 30, 130. **Beside to what above, note that:**
- One, or more than two, fastening holes may be provided in each of the talar and tibial implants.
- Fastening holes 50, 70, 150, 170, and the heads of fasteners 40, 60, may be cylindrical instead of frustoconical, but otherwise be identical to the fasteners and fastening holes described herein.
- The fasteners and fastening holes may be completely unthreaded and lock by a taper lock resulting from the complementary frustoconical shape of the fastener heads (as shown in Figures 28A to 28C) and fastening holes. As the fastener would be impacted in for locking, no feature is necessary on the head of the fastener for mating connection with a screwdriver or drill. Otherwise, the taper locked fasteners and fastening holes are identical to the screw lock fasteners and fastening holes described herein. A taper locked fastener/fastening hole system may utilize neutral locking or compression locking as described herein. For example, for compression locking, the drill hole may be drilled eccentrically toward the side of the unthreaded fastening hole in which it is desired for the implant to be pulled.
- The compression locking system described herein may be used in contexts of joints other than the ankle including knee, hip, shoulder, elbow and wrist.

## Claims

1. An ankle prosthesis comprising:
a talar implant (20, 120, 220, 320) comprising a body (21, 121, 221, 321) for mounting to a top of a talus, the body comprising at least one **frustoconical** talar fastening hole (26, 26', 126, 126', 226, 326);
a tibial implant (30, 130) comprising a plate (31, 131) for mounting to a bottom of a tibia;
a flange (34, 134) extending from the plate for bearing against an anterior surface of the tibia, the flange comprising at least one **frustoconical** tibial fastening hole (36, 36', 136, 136');
a mobile bearing for positioning between the talar implant and the tibial implant; and
a plurality of fasteners (28, 28', 38, 38', 128, 128', 138, 138', 228, 328) for compression locking engagement with the at least one **frustoconical** talar fastening hole and the at least one **frustoconical** tibial fastening hole for fastening the talar implant and the tibial implant to the talus and the tibia respectively, each of the fasteners comprising locking means and an elongated unthreaded body;
**characterized in that** the at least one **frustoconical** talar fastening hole and the at least one **frustoconical** tibial fastening hole each comprise a distal end comprising:
a **threaded** arc section (72, 172) having a first radius; and
a **unthreaded** arc section (76, 176) having a second radius greater than the first radius;
whereby the fastener is eccentrically insertable through the at least one **frustoconical** talar fastening hole and the at least one **frustoconical** tibial fastening hole, the eccentricity defined by a shift of the fastener toward the **unthreaded** arc section by a distance of up to the difference between the first and the second radii.

2. An ankle prostheses according to claim 1, wherein the **unthreaded** arc section is situated on an anterior side of the at least one **frustoconical** talar fastening hole.

3. An ankle prostheses according to claim 1 or 2, wherein the **unthreaded** arc section is situated on an upper side of the at least one **frustoconical** tibial fastening hole.

4. An ankle prosthesis according to any of claims 1 to 3 wherein the **frustoconical** talar fastening holes are configured for vertical insertion of the fasteners and the **frustoconical** tibial fastening holes are configured for horizontal insertion of the fasteners.

5. An ankle prosthesis according to any of claims 1 to 4, wherein the at least one **frustoconical** talar fastening hole is configured for insertion of the fastener at an acute posterior angle and the at least one **frustoconical** tibial fastening hole is configured for insertion of the fastener at an acute upward angle.

6. An ankle prosthesis according to claim 5, wherein the acute posterior angle is substantially the same as the angle at which a posterior component of the body extends downward from the horizontal.

7. An ankle prosthesis according to any of claims 1 to 6, wherein the **threaded** arc section comprises internal threads (74, 174), the **unthreaded** arc section comprises an unthreaded section, and the locking means comprise external threads on a head of each fastener for screw locking of the fasteners to the at least one **frustoconical** talar fastening hole and the at least one **frustoconical** tibial fastening hole.

8. An ankle prosthesis according to claim 7, wherein the elongated unthreaded body of each of the fasteners comprises a smooth surface (65) and a non-smooth surface (67), wherein the fastener is insertable with the smooth surface facing the unthreaded section.

9. An ankle prosthesis according to claim 1 to 5, wherein the at least one **frustoconical** talar fastening hole and the at least one **frustoconical** tibial fastening hole are frustoconical and the locking means comprise a frustoconical head for taper locking of each fastener to the at least one **frustoconical** talar fastening hole and the at least one **frustoconical** tibial fastening hole.

10. An ankle prosthesis according to any of claims 1 to 9, wherein the body of the talar implant comprises an anterior flange (24, 124, 224) extending from the body for bearing against a neck of the talus.

11. An ankle prosthesis according to claim 10, wherein the anterior flange comprises the at least one **frustoconical** talar fastening hole.

12. An ankle prosthesis according to any of claims 1 to 11, wherein the talar implant comprises an upper anterior surface comprising the at least one **frustoconical** talar fastening hole.

13. An ankle prosthesis according to any of claims 1 to 12 comprising two **frustoconical** talar fastening holes and two **frustoconical** tibial fastening holes.

14. An ankle prosthesis according to any of claims 1 to 13, wherein the **frustoconical** tibial fastening holes are configured for insertion of the fasteners in a direction parallel to the medial border of the tibia in the transverse plane.

## Patentansprüche

1. Sprunggelenksprothese, umfassend:
ein Talus-Implantat (20, 120, 220, 320), das einen Körper (21, 121, 221, 321) für die Befestigung an einer Oberseite eines Talus umfasst, wobei der Körper mindestens ein kegelstumpfförmiges Talus-Befestigungsloch (26, 26', 126, 126', 226, 326) umfasst;
ein Tibia-Implantat (30, 130), das eine Platte (31, 131) für die Befestigung an einer Unterseite einer Tibia umfasst;
einen Flansch (34, 134), der sich von der Platte zur Abstützung gegen eine vordere Oberfläche der Tibia erstreckt, wobei der Flansch mindestens ein kegelstumpfförmiges Tibia-Befestigungsloch (36, 36', 136, 136') umfasst;
ein bewegliches Lager zum Positionieren zwischen dem Talus-Implantat und dem Tibia-Implantat; und
eine Vielzahl von Befestigungselementen (28, 28', 38, 38', 128, 128', 138, 138', 228, 328) für den Kompressionsverriegelungseingriff mit dem mindestens einen kegelstumpfförmigen Talus-Befestigungsloch und dem mindestens einen kegelstumpfförmigen Tibia-Befestigungsloch für die Befestigung des Talus-Implantats und des Tibia-Implantats am Talus beziehungsweise an der Tibia, wobei jedes der Befestigungselemente Verriegelungsmittel und einen länglichen gewindelosen Körper umfasst;
**dadurch gekennzeichnet, dass** das mindestens eine kegelstumpfförmige Talus-Befestigungsloch und das mindestens eine kegelstumpfförmige Tibia-Befestigungsloch jeweils ein distales Ende umfassen, das Folgendes umfasst:
einen gewindeten Bogenabschnitt (72, 172) mit einem ersten Radius; und
einen gewindelosen Bogenabschnitt (76, 176) mit einem zweiten Radius, der größer als der erste Radius ist;
wobei das Befestigungselement exzentrisch durch das mindestens eine kegelstumpfförmige Talus-Befestigungsloch und das mindestens eine kegelstumpfförmige Tibia-Befestigungsloch eingefügt werden kann, wobei die Exzentrizität durch eine Verschiebung des Befestigungselements zum gewindelosen Bogenabschnitt um eine Strecke definiert wird, die maximal der Differenz zwischen dem ersten und dem zweiten Radius entspricht.

2. Sprunggelenksprothese nach Anspruch 1, bei der sich der gewindelose Bogenabschnitt auf einer Vorderseite des mindestens einen kegelstumpfförmigen Talus-Befestigungslochs befindet.

3. Sprunggelenksprothese nach Anspruch 1 oder 2, bei der sich der gewindelose Bogenabschnitt auf einer Oberseite des mindestens einen kegelstumpfförmigen Tibia-Befestigungslochs befindet.

4. Sprunggelenksprothese nach einem der Ansprüche 1 bis 3, bei der die kegelstumpfförmigen Talus-Befestigungslöcher zum senkrechten Einfügen der Befestigungselemente gestaltet sind und die kegelstumpfförmigen Tibia-Befestigungslöcher zum waagrechten Einfügen der Befestigungselemente gestaltet sind.

5. Sprunggelenksprothese nach einem der Ansprüche 1 bis 4, bei der das mindestens eine kegelstumpfförmige Talus-Befestigungsloch zum Einfügen des Befestigungselements in einem spitzen posterioren Winkel gestaltet ist und das mindestens eine kegelstumpfförmige Tibia-Befestigungsloch zum Einfügen des Befestigungselements in einem spitzen Winkel nach oben gestaltet ist.

6. Sprunggelenksprothese nach Anspruch 5, bei welcher der spitze posteriore Winkel im Wesentlichen gleich dem Winkel ist, mit dem sich eine hintere Komponente des Körpers von der Waagrechten nach unten erstreckt.

7. Sprunggelenksprothese nach einem der Ansprüche 1 bis 6, bei welcher der gewindete Bogenabschnitt Innengewinde (74, 174) umfasst, der gewindelose Bogenabschnitt einen gewindelosen Abschnitt umfasst und die Verriegelungsmittel Außengewinde auf einem Kopfstück von jedem Befestigungselement für die Schraubverriegelung der Befestigungselemente an dem mindestens einen kegelstumpfförmigen Talus-Befestigungsloch und dem mindestens einen kegelstumpfförmigen Tibia-Befestigungsloch umfassen.

8. Sprunggelenksprothese nach Anspruch 7, bei welcher der längliche gewindelose Körper von jedem der Befestigungselemente eine glatte Oberfläche (65) und eine nicht glatte Oberfläche (67) umfasst, wobei das Befestigungselement mit der dem gewindelosen Abschnitt zugewandten glatten Oberfläche eingefügt werden kann.

9. Sprunggelenksprothese nach den Ansprüchen 1 bis 5, bei der das mindestens eine kegelstumpfförmige Talus-Befestigungsloch und das mindestens eine kegelstumpfförmige Tibia-Befestigungsloch kegelstumpfförmig sind und die Verriegelungsmittel ein kegelstumpfförmiges Kopfstück für die Konusverriegelung von jedem Befestigungselement an dem mindestens einen kegelstumpfförmigen Talus-Befestigungsloch und dem mindestens einen kegelstumpfförmigen Tibia-Befestigungsloch umfassen.

10. Sprunggelenksprothese nach einem der Ansprüche 1 bis 9, bei welcher der Körper des Talus-Implantats einen vorderen Flansch (24, 124, 224) umfasst, der sich von dem Körper zur Abstützung gegen einen Hals des Talus erstreckt.

11. Sprunggelenksprothese nach Anspruch 10, bei welcher der vordere Flansch mindestens ein kegelstumpfförmiges Talus-Befestigungsloch umfasst.

12. Sprunggelenksprothese nach einem der Ansprüche 1 bis 11, bei der das Talus-Implantat eine obere vordere Oberfläche umfasst, die mindestens ein kegelstumpfförmiges Talus-Befestigungsloch umfasst.

13. Sprunggelenksprothese nach einem der Ansprüche 1 bis 12, die zwei kegelstumpfförmige Talus-Befestigungslöcher und zwei kegelstumpfförmige Tibia-Befestigungslöcher umfasst.

14. Sprunggelenksprothese nach einem der Ansprüche 1 bis 13, bei der die kegelstumpfförmigen Tibia-Befestigungslöcher zum Einfügen der Befestigungselemente in einer Richtung gestaltet sind, die parallel zum medialen Rand der Tibia in der Querebene ist.

## Revendications

1. Prothèse de cheville comprenant :
un implant talien (20, 120, 220, 320) comprenant un corps (21, 121, 221, 321) pour le montage sur un dessus d'un talus, le corps comprenant au moins un trou de fixation tronconique talien (26, 26', 126, 126', 226, 326) ;
un implant tibial (30, 130) comprenant une plaque (31, 131) pour le montage sur un dessous d'un tibia ;
une bride (34, 134) s'étendant à partir de la plaque pour l'appui contre une surface antérieure du tibia, la bride comprenant au moins un trou de fixation tronconique tibial (36, 36', 136, 136') ;
un insert mobile pour le positionnement entre l'implant talien et l'implant tibial ; et
une pluralité d'éléments de fixation (28, 28', 38, 38', 128, 128', 138, 138', 228, 328) pour l'engagement de blocage par compression avec l'au moins un trou de fixation tronconique talien et l'au moins un trou de fixation tronconique tibial pour la fixation de l'implant talien et de l'implant tibial respectivement au talus et au tibia, chacun des éléments de fixation comprenant des moyens de blocage et un corps allongé non fileté ;
**caractérisée en ce que** l'au moins un trou de fixation tronconique talien et l'au moins un trou de fixation tronconique tibial comprennent chacun une extrémité distale comprenant :
une section d'arc filetée (72, 172) ayant un premier rayon ; et
une section d'arc non filetée (76, 176) ayant un deuxième rayon supérieur au premier rayon ;
moyennant quoi l'élément de fixation peut être inséré de manière excentrique à travers l'au moins un trou de fixation tronconique talien et l'au moins un trou de fixation tronconique tibial, l'excentricité étant définie par un décalage de l'élément de fixation vers la section d'arc non filetée d'une distance pouvant aller jusqu'à la différence entre le premier et le deuxième rayons.

2. Prothèse de cheville selon la revendication 1, dans laquelle la section d'arc non filetée est située sur un côté antérieur de l'au moins un trou de fixation tronconique talien.

3. Prothèse de cheville selon la revendication 1 ou 2, dans laquelle la section d'arc non filetée est située sur un côté supérieur de l'au moins un trou de fixation tronconique tibial.

4. Prothèse de cheville selon l'une quelconque des revendications 1 à 3, dans laquelle les trous de fixation tronconiques taliens sont configurés pour l'insertion verticale des éléments de fixation et les trous de fixation tronconiques tibiaux sont configurés pour l'insertion horizontale des éléments de fixation.

5. Prothèse de cheville selon l'une quelconque des revendications 1 à 4, dans laquelle l'au moins un trou de fixation tronconique talien est configuré pour l'insertion de l'élément de fixation à un angle aigu postérieur et l'au moins un trou de fixation tronconique tibial est configuré pour l'insertion de l'élément de fixation à un angle aigu vers le haut.

6. Prothèse de cheville selon la revendication 5, dans laquelle l'angle aigu postérieur est sensiblement le même que l'angle avec lequel un composant postérieur du corps s'étend vers le bas par rapport à l'horizontale.

7. Prothèse de cheville selon l'une quelconque des revendications 1 à 6, dans laquelle la section d'arc filetée comprend des filets internes (74, 174), la section d'arc non filetée comprend une section non filetée, et les moyens de blocage comprennent des filets externes sur une tête de chaque élément de fixation pour le blocage par vissage des éléments de fixation à l'au moins un trou de fixation tronconique talien et l'au moins un trou de fixation tronconique tibial.

8. Prothèse de cheville selon la revendication 7, dans laquelle le corps allongé non fileté de chacun des éléments de fixation comprend une surface lisse (65) et une surface non lisse (67), dans lequel l'élément de fixation peut être inséré avec la surface lisse faisant face à la section non filetée.

9. Prothèse de cheville selon l'une quelconque des revendications 1 à 5, dans laquelle l'au moins un trou de fixation tronconique talien et l'au moins un trou de fixation tronconique tibial sont tronconiques et les moyens de blocage comprennent une tête tronconique pour le blocage conique de chaque élément de fixation avec l'au moins un trou de fixation tronconique talien et l'au moins un trou de fixation tronconique tibial.

10. Prothèse de cheville selon l'une quelconque des revendications 1 à 9, dans laquelle le corps de l'implant talien comprend une bride antérieure (24, 124, 224) s'étendant à partir du corps pour être en appui contre un col de l'astragale.

11. Prothèse de cheville selon la revendication 10, dans laquelle la bride antérieure comprend l'au moins un trou de fixation tronconique talien.

12. Prothèse de cheville selon l'une quelconque des revendications 1 à 11, dans laquelle l'implant talien comprend une surface antérieure supérieure comprenant l'au moins un trou de fixation tronconique talien.

13. Prothèse de cheville selon l'une quelconque des revendications 1 à 12, comprenant deux trous de fixation tronconiques taliens et deux trous de fixation tronconiques tibiaux.

14. Prothèse de cheville selon l'une quelconque des revendications 1 à 13, dans laquelle les trous de fixation tronconiques tibiaux sont configurés pour l'insertion des éléments de fixation dans une direction parallèle au bord médial du tibia dans le plan transversal.
